# EUROPEAN PATENT APPLICATION

(11) **EP 4 850 670 A1**
(43) Date of publication of application: **09.12.2026**
(21) Application number: 25751340.8
(22) Date of filing: 24.01.2025
(51) Int. Cl.: A61K 31/437, A61P 7/06, A61P 35/00

(54) **USE OF COMPOUND IN PREPARING MEDICAMENT FOR TREATING ANEMIA IN NON-MYELOID MALIGNANT TUMOR PATIENT**

(30) Priority: 05.02.2024 CN 202410169767; 07.05.2024 CN 202410553582
(71) Applicant: Shenzhen Salubris Pharmaceuticals Co., Ltd., Shenzhen, Guangdong 518017 (CN)
(72) Inventor: XIAO, Ying, Shenzhen, Guangdong 518017 (CN); MU, Shengmei, Shenzhen, Guangdong 518017 (CN); DUAN, Zhenfang, Shenzhen, Guangdong 518017 (CN); CHEN, Yuanyuan, Shenzhen, Guangdong 518017 (CN); XING, Yan, Shenzhen, Guangdong 518017 (CN); LIU, Zichen, Shenzhen, Guangdong 518017 (CN); LIN, Wei, Shenzhen, Guangdong 518017 (CN)
(74) Representative: Gassner, Birgitta
(86) International application number: PCT/CN2025/074692
(87) International publication number: WO 2025/167738

(57) **Abstract**

Provided is use of a compound and a pharmaceutical composition thereof in preparing a medicament for treating anemia in a non-myeloid malignant tumor patient receiving chemotherapy, wherein the compound is N-[7-hydroxy-5-(2-phenylethyl)[1,2,4]triazole[1,5-a]pyridine-8-carbonyl]glycine.

## Description

### TECHNICAL FIELD

The present disclosure belongs to the technical field of medical use, and relates to use of a compound and a pharmaceutical composition thereof in preparation of a medicament for treating anemia in a patient with a non-myeloid malignancy who is undergoing chemotherapy, where the compound is *N*-[7-hydroxy-5-(2-phenylethyl)[1,2,4]triazolo[1,5-a]pyridine-8-carbonyl]glycine.

### BACKGROUND

CN102471337B discloses a triazolopyridine compound having a prolyl hydroxylase inhibitory effect and erythropoietin production-inducing capability, specifically including a compound with a structure of the following formula:

### Chemical name: N-[7-hydroxy-5-(2-phenylethyl)[1,2,4]triazolo[1,5-a]pyridine-8-carbonyl]glycine

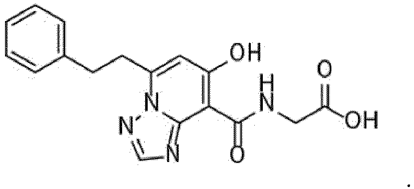

Anemia is prevalent in a patient with a non-myeloid malignancy who is undergoing chemotherapy. Currently, there is a lack of relatively effective oral medications for this patient population. Myeloid malignancies can be classified according to relevant World Health Organization (WHO) classifications. Myeloid malignancies other than these myeloid malignancies are classified as non-myeloid malignancies.

### SUMMARY

In view of the problems existing in the prior art, the present disclosure provides use of a compound in preparation of a medicament for treating anemia in a patient with a non-myeloid malignancy who is undergoing chemotherapy (also referred to as "chemotherapy-induced anemia in a patient with a non-myeloid malignancy"), wherein the compound is *N*-[7-hydroxy-5-(2-phenylethyl)[1,2,4]triazolo[1,5-a]pyridine-8-carbonyl]glycine (Compound A).

The *N*-[7-hydroxy-5-(2-phenylethyl)[1,2,4]triazolo[1,5-a]pyridine-8-carbonyl]glycine is a compound with a structure of the following formula:

### Chemical name: N-[7-hydroxy-5-(2-phenylethyl)[1,2,4]triazolo[1,5-a]pyridine-8-carbonyl]glycine

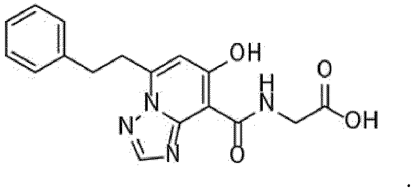

The present disclosure further provides use of a pharmaceutical composition comprising the compound in preparation of a medicament for treating anemia in a patient with a non-myeloid malignancy who is undergoing chemotherapy, where the compound is *N*-[7-hydroxy-5-(2-phenylethyl)[1,2,4]triazolo[1,5-a]pyridine-8-carbonyl]glycine, and the pharmaceutical composition comprises one or more excipients.

As a preferred technical scheme of the present disclosure, the daily dose is about 1 mg to about 30 mg, including:
1 mg, 1.1 mg, 1.2 mg, 1.3 mg, 1.4 mg, 1.5 mg, 1.6 mg, 1.7 mg, 1.8 mg, 1.9 mg; 2 mg, 2.1 mg, 2.2 mg, 2.3 mg, 2.4 mg, 2.5 mg, 2.6 mg, 2.7 mg, 2.8 mg, 2.9 mg; 3 mg, 3.1 mg, 3.2 mg, 3.3 mg, 3.4 mg, 3.5 mg, 3.6 mg, 3.7 mg, 3.8 mg, 3.9 mg; 4 mg, 4.1 mg, 4.2 mg, 4.3 mg, 4.4 mg, 4.5 mg, 4.6 mg, 4.7 mg, 4.8 mg, 4.9 mg; 5 mg, 5.1 mg, 5.2 mg, 5.3 mg, 5.4 mg, 5.5 mg, 5.6 mg, 5.7 mg, 5.8 mg, 5.9 mg; 6 mg, 6.1 mg, 6.2 mg, 6.3 mg, 6.4 mg, 6.5 mg, 6.6 mg, 6.7 mg, 6.8 mg, 6.9 mg; 7 mg, 7.1 mg, 7.2 mg, 7.3 mg, 7.4 mg, 7.5 mg, 7.6 mg, 7.7 mg, 7.8 mg, 7.9 mg; 8 mg, 8.1 mg, 8.2 mg, 8.3 mg, 8.4 mg, 8.5 mg, 8.6 mg, 8.7 mg, 8.8 mg, 8.9 mg; 9 mg, 9.1 mg, 9.2 mg, 9.3 mg, 9.4 mg, 9.5 mg, 9.6 mg, 9.7 mg, 9.8 mg, 9.9 mg; 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, and 30 mg.

As a preferred technical scheme of the present disclosure, the patient is undergoing platinum-based myelosuppressive chemotherapy.

As a preferred technical scheme of the present disclosure, the patient is undergoing non-platinum-based myelosuppressive chemotherapy.

As a preferred technical scheme of the present disclosure, the chemotherapy is myelosuppressive chemotherapy, and a myelosuppressive chemotherapy agent includes:
Atezolizumab, Bevacizumab, Reslizumab, Cetuximab, Denosumab, Durvalumab, Envafolimab, Inetetamab, Pembrolizumab, Penpulimab, Tislelizumab, Sintilimab, Toripalimab;
Gefitinib, Cabozantinib, Pyrotinib, Apatinib;
Carboplatin, Cisplatin, Lobaplatin, Nedaplatin, Oxaliplatin;
Paclitaxel, Docetaxel, Capecitabine, Gemcitabine, Cyclophosphamide, Epirubicin, Eribulin, Etoposide, Fluorouracil, Fulvestrant, Irinotecan, Doxorubicin, Cantharidin, Oteracil, Pemetrexed, Pyridoxine, Tamoxifen, Tegafur, and Vinorelbine.

As a preferred technical scheme of the present disclosure, the non-myeloid malignancy includes breast cancer, lung cancer, gastrointestinal cancer, gynecological cancer, pancreatic cancer, and head and neck cancer.

As a preferred technical scheme of the present disclosure, the lung cancer includes small cell lung cancer and non-small cell lung cancer; the gastrointestinal cancer includes gastric cancer, colon cancer, and rectal cancer; and the gynecological cancer includes cervical cancer, endometrial cancer, and ovarian cancer.

As a preferred technical scheme of the present disclosure, the platinum-based chemotherapy includes the use of a drug for a non-myeloid malignancy, such as carboplatin and cisplatin, etc., wherein:
Cisplatin is primarily used for the treatment of testicular tumors and osteosarcoma. Cisplatin exhibits a certain therapeutic efficacy against ovarian cancer, breast cancer, lung cancer, head and neck cancer, bladder cancer, malignant lymphoma, esophageal cancer, gastric cancer, and cervical cancer.

Carboplatin is primarily used for indications including small cell lung cancer, ovarian cancer, testicular cancer, head and neck squamous cell carcinoma, bladder cancer, pleural mesothelioma, cervical cancer, and non-small cell lung cancer.

As an implementation technical scheme of the present disclosure, the preparation of the compound is incorporated herein by reference to the technical scheme described in CN201780072928.2.

The crystal form and preparation used in the pharmaceutical composition are incorporated herein by reference to the technical scheme described in US20200017492A1.

The beneficial effects of the present disclosure over the prior art include, but are not limited to:
(1) use of the compound of the present disclosure in preparation of a medicament for treating anemia in a patient with a non-myeloid malignancy who is undergoing chemotherapy is superior to Roxadustat in terms of lower dosage and reduced adverse effects, and the like. However, for animals with myeloid malignancies, no significant improvement is observed in the experimental results.

### BRIEF DESCRIPTION OF DRAWINGS

FIG. 1 shows a clinical trial procedure for Enarodustat (Compound A) tablets in Example 3.
FIG. 2 shows a clinical dose adjustment protocol for Enarodustat (Compound A) tablets in Example 3.
FIG. 3 shows Hemoglobin (Hb) test results from a central laboratory.
FIG. 4 shows Hematocrit (Hct) test results from a local laboratory.
FIG. 5 shows platelet counts in peripheral blood of rats in Example 4.
FIG. 6 shows red blood cell counts in peripheral blood of rats in Example 4.

### DETAILED DESCRIPTION

The present disclosure will be further described in detail hereafter in combination with examples, but the embodiments of the present disclosure are not limited thereto.

### Experiment 1: Study of Therapeutic Effect of Compound A on Carboplatin-Induced Anemia Model in Rats

Test Method: Male Sprague-Dawley (SD) rats (200-230 g) were used. After the completion of quarantine and acclimatization, the rats were randomly assigned to groups based on hematocrit levels. The groups were as follows: a normal control group, a model control group, a Roxadustat group (40 mg/kg), and low-dose group of Compound A (1 mg/kg), medium-dose group of Compound A (3 mg/kg), and high-dose group of Compound A (10 mg/kg), with 10 rats per group.

On Day 1 (D1) of the experiment, the normal control group was intraperitoneally administered with a vehicle, while the other groups were intravenously administered with carboplatin (45 mg/kg) for modeling. Two hours before modeling, the Roxadustat group was received Roxadustat (40 mg/kg) by gavage, the respective Compound A groups were received Compound A (1, 3, or 10 mg/kg) by gavage, and the model control group was received the vehicle by gavage. Thereafter, the Roxadustat treatment group was received continued administration by gavage on days 3, 5, 8, 10, and 12, and Compound A groups were administered once daily for 14 consecutive days.

Measured parameters included: whole blood was collected on Day 14 (D14) post-initial dosing, and anticoagulation was performed with EDTA-K₂, and the hematological indicators such as red blood cell count (RBC), hematocrit (HCT), and hemoglobin concentration (Hb) were determined within 1 h after blood collection using an automated hematology analyzer. The results are shown in Table 1.

**Table 1**

| Groups | Day 14 | | |
|---|---|---|---|
| | RBC (10¹²/L) | HGB (g/L) | Hct (%) |
| Normal control group | 7.67 ± 0.29 | 164.9 ± 6.28 | 48.76 ± 2.18 |
| Model control group | 5.58 ± 1.15 | 128.6 ± 24.96 | 35.75 ± 7.75 |
| Roxadustat-40 mg/kg | 5.12 ± 1.88 | 127.78 ± 46.58 | 34.13 ± 12.92 |
| Compound A-1 mg/kg | 5.89 ± 0.91 | 136.6 ± 16.17 | 37.85 ± 5.77 |
| Compound A-3 mg/kg | 5.57 ± 0.97 | 133.3 ± 23.53 | 36.34 ± 6.8 |
| Compound A-10 mg/kg | 6.85 ± 0.92 | 166.6 ± 21.76 | 49.45 ± 8.37 |

Results: The results on Day 14 showed that the indicators including RBC, Hct and Hb of the animals in the Compound A (1 mg/kg, 3 mg/kg, and 10 mg/kg) treatment groups were significantly improved, and were superior to the Roxadustat group.

### Experiment 2: Study of Therapeutic Effect of Compound A on Cisplatin-Induced Anemia Model in Rats

Test Method: Male Wistar rats (200-230 g) were used. After the completion of quarantine and acclimatization, the rats were randomly assigned to groups based on hematocrit levels. The groups were as follows: a normal control group, a model control group, and low-, medium-, and high-dose groups of Compound A (1 mg/kg, 3 mg/kg, and 10 mg/kg), with 10 rats per group.

On Day 1 (D1) of the experiment, the normal control group was intraperitoneally administered with a vehicle, while the other groups were intravenously administered with cisplatin (5 mg/kg) for modeling. Two hours before modeling, the respective Compound A groups were received Compound A (1, 3, or 10 mg/kg) by gavage, and the model control group was received the vehicle by gavage, and Compound A groups were administered once daily for 14 consecutive days.

Measured parameters: whole blood was collected on Day 14 (D14) post-initial dosing, and anticoagulation was performed with EDTA-K₂, and the hematological indicators such as red blood cell count (RBC), hematocrit (HCT), and hemoglobin concentration (Hb) were determined within 1 h after blood collection using an automated hematology analyzer.

Results: The results on Day 14 showed that the indicators including RBC, Hct and Hb of the animals in the Compound A (1 mg/kg, 3 mg/kg, and 10 mg/kg) treatment groups were significantly improved.

### Example 3:

### Test Objective:

For evaluation of Safety and efficacy of Enarodustat (Compound A) tablets in the treatment of chemotherapy-induced anemia in a patient with a non-myeloid malignancy, and their effects on red blood cell-related and iron metabolism-related test values, as well as pharmacokinetic characteristics

### Test drug:

Enarodustat, 1 mg, 2 mg, 4 mg, and 5 mg, tablets

### Test Procedures:

The test included a screening period (4 weeks), an open-label treatment period (up to 16 weeks), and a follow-up period (2 weeks), lasting approximately 22 weeks in total.

### Dosing Regimen:

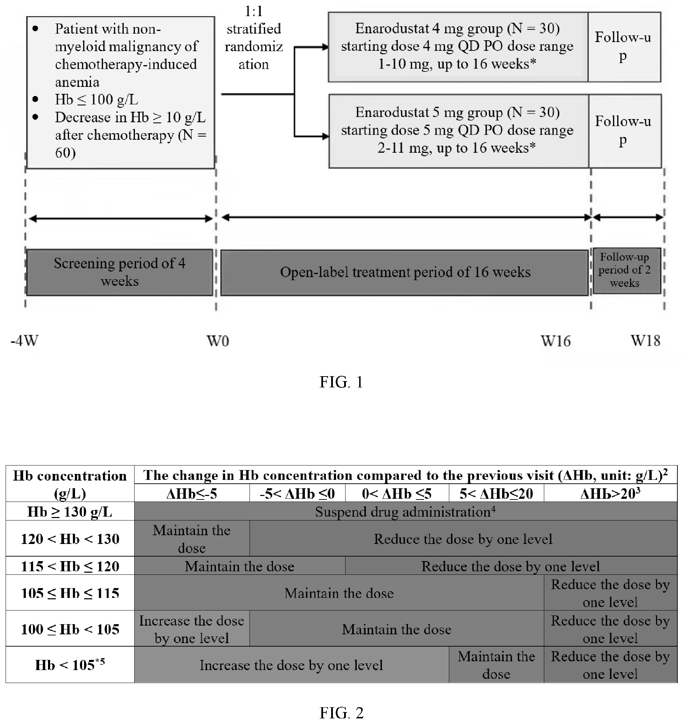

The subjects were grouped according to the starting dose into a 4 mg starting dose group and a 5 mg starting dose group. Every 4 weeks, individualized dose adjustments were made based on the Hb levels of the subjects to maintain Hb levels at ≥ 100 and < 120 g/L. The test flowchart and dose adjustment rules were shown in FIG. 1 and FIG. 2, respectively:
- 4 mg Starting Dose Group: the doses were adjusted stepwise among 1 mg, 2 mg, 4 mg, 6 mg, 8 mg, and 10 mg; skipping dose levels was not permitted.
- 5 mg Starting Dose Group: the doses were adjusted stepwise among 2 mg, 3 mg, 5 mg, 7 mg, 9 mg, and 11 mg; skipping dose levels was not permitted.
- Administered orally once daily, either before a meal or at bedtime, for a maximum of 16 consecutive weeks. Study treatment should be discontinued upon completion of the subject's entire chemotherapy course.
- Definition of Pre-meal Dosing: take the medication at least 1 hour before the next meal and at least 2 hours after the previous meal. It is recommended to take the medication before the same meal each day.

### Inclusion Criteria:

Subjects are eligible for enrollment if they meet all of the following criteria:
1. Chinese male and female subjects over 18 years of age, who are at least 18 years old (including the date of their 18th birthday) at the time of signing the Informed Consent Form (ICF);
2. Body weight ≥ 40 kg during the screening period;
3. Have a histologically or cytologically confirmed diagnosis of non-myeloid malignancy (with non-curative intent), and are planned to receive at least 8 weeks of anti-tumor therapy (myelosuppressive chemotherapy) from the first administration day (Day 1);
4. Have myelosuppressive chemotherapy-related anemia, defined as hemoglobin (Hb) ≤ 100 g/L by central laboratory assessment during the screening period, with documented evidence of a ≥ 10 g/L decrease in Hb levels after chemotherapy initiation, as judged by the investigator;
5. Ferritin ≥ 50 ng/mL and transferrin saturation (TSAT) ≥ 10% from the laboratory during the screening period;
6. Eastern Cooperative Oncology Group (ECOG) performance status score ≤ 1 during the screening period;
7. Life expectancy ≥ 6 months as determined by the investigator during the screening period;
8. All male subjects and female subjects of childbearing potential agree to use medically acceptable contraceptive methods from the date of ICF signature until 90 days after the last dose of the test drug;
9. Are willing to participate and have signed the Informed Consent Form (ICF), are able to understand the procedures and methods of this test and are willing to strictly adhere to the clinical trial protocol to complete the test.

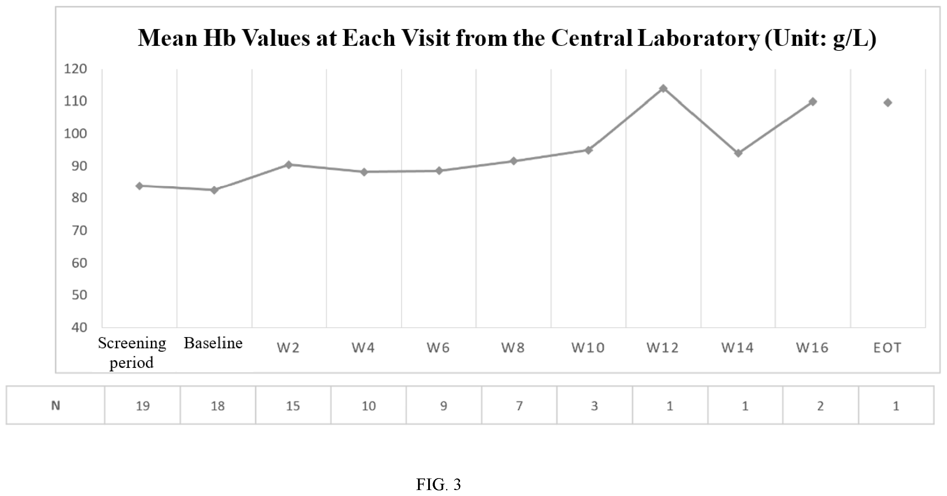

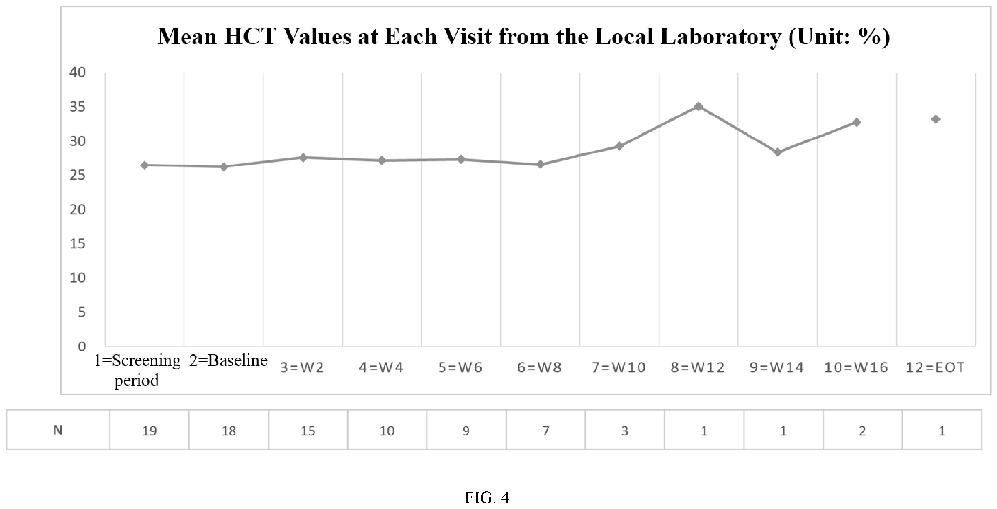

The research found that 5 weeks after administration, Hb increases of > 5 g/L were observed in some patients. Central laboratory Hb and local laboratory Hct test results are shown in FIG. 3 and FIG. 4, respectively, with both Hb and Hct showing certain improvements.

### Example 4: Therapeutic Effect of Compound A on Chemotherapy-Induced Anemia

Human hepatocellular carcinoma cell line HUH-7 was used in this experiment. After expansion and culture, tumor cells in the logarithmic growth phase were harvested and used for tumor inoculation in mice. The cells were collected, washed twice with phosphate-buffered saline (PBS), and adjusted to a cell suspension of an appropriate density for subsequent use. The cell suspension was injected subcutaneously at 100 µL per mouse into the left flank of BALB/c nude mice, the number of cells injected was 2 × 10⁶ cells/mouse. Mice were randomized into groups for administration based on tumor volume and body weight of the mice when the tumor grew to an average tumor volume of approximately 100-120 mm³ (the day of grouping was designated as Day 0), and administration began on the day after the grouping (Day 1). Grouping and administration are shown in Table 2.

**Table 2: Grouping and Administration Table**

| **Groups** | Number of animals/group | **Drug dose (mg/kg)** | **Administration volume (mL/kg)** | **Administration route** | **Cycle** |
|---|---|---|---|---|---|
| Vehicle Control Group | 11 | 0 + 0 | 10 + 10 | IP + PO | Q2W + QD |
| Carboplatin Monotherapy Group | 11 | 100 + 0 | 10 + 10 | IP + PO | Q2W + QD |
| Carboplatin + Compound A Group | 11 | 100 + 60 | 10 + 10 | IP + PO | Q2W + QD |
| Carboplatin + Roxadustat Group | 11 | 100 + 60 | 10 + 10 | IP + PO | Q2W + QD |

In the vehicle control group, mice were received vehicle via both intraperitoneal injection and gavage. In the carboplatin monotherapy group, mice were administered with carboplatin (100 mg/kg) via intraperitoneal injection once every two weeks, and were concurrently received the vehicle once daily by gavage; in the combination therapy group, animals were received carboplatin (100 mg/kg) via intraperitoneal injection, and were concurrently administered compound A (60 mg/kg) or Roxadustat (60 mg/kg), respectively, once daily by gavage. The entire experimental period lasted for 20 days, during which carboplatin was administered twice, and compound A and Roxadustat were each administered 20 times.

After administration, 50 µL of whole blood was collected via retro-orbital bleeding on Day 8, Day 15, and Day 20, respectively, and placed in EDTA-K2 anticoagulant tubes, and red blood cell (RBC) count, hemoglobin (Hb), and hematocrit (Hct) were measured using an automated hematology analyzer. At the end of the experiment, animals were euthanized. The results are shown in Table 3.

**Table 3: Cell Parameter Values of Mice During Administration**

| Groups | Hematological Indicators | | | | | | | | |
|---|---|---|---|---|---|---|---|---|---|
| | RBC (10¹²/L) | | | HGB (g/L) | | | HCT (%) | | |
| | Day 8 | Day 15 | Day 20 | Day 8 | Day 15 | Day 20 | Day 8 | Day 15 | Day 20 |
| Vehicle Control Group | 10.7 4 ± 1.2 | 9.9 ± 2.27 | 10.79 ± 1.25 | 169.09 ± 13.03 | 154.82 ± 34.59 | 170.4 ± 18.97 | 54.15 ± 3.95 | 51.32 ± 11.96 | 56.22 ± 6.62 |
| Carboplatin Monotherapy Group | 11.5 9 ± 1.61 | 7.51 ± 1.09 | 7.77 ± 0.99 | 181.45 ± 22.92 | 120.55 ± 16.08 | 134.55 ± 11.54 | 56.38± 7.56 | 37.25 ± 5.38 | 41.06 ± 4.08 |
| Carboplatin+C | 12.4 | 10.6 | 11.23 ± | 203.64 ± | 180.55 ± | 199.55 ± | 62.39 ± | 57.56 ± | 62.89 ± |
| ompound A Group | 6 ± 0.85 | 8 ± 1.41 | 1.43 | 15.13 | 13.34 | 17.08 | 5.3 | 5.12 | 4.98 |
| Carboplatin+R oxadustat Group | 10.6 1 ± 1.66 | 9.03 ± 0.88 | 8.03 ± 1.03 | 177.55 ± 22.81 | 159.6 ± 13.9 | 156.7 ± 19 | 53.17 ± 7.7 | 48.69 ± 4.94 | 44.69 ± 5.84 |

The results showed that the red blood cell parameters in tumor-bearing mice significantly decreased over time after the administration of carboplatin, indicating that the chemotherapeutic agent induced anemia in the subcutaneous hepatocellular carcinoma xenograft mice. After the administration of compound A or Roxadustat, respectively, RBC, Hb, and Hct levels were significantly improved. At an equivalent dose, compound A showed greater improvement compared to Roxadustat.

### Example 5: Therapeutic Effect of Compound A on Myelodysplastic Syndrome in Rat Model

### 1. Methods

4 to 5-week-old Sprague-Dawley (SD) rats were used. The rats were randomized into a normal control group and a group for modeling. Before modeling induction, blood samples were collected from all experimental animals and the initial hematological values were assessed.

Modeling: on Day 0, animals were received the first induction via tail vein injection (i.v.) of 35 mg/kg of a dimethyl benzanthracene (DMBA) modeling agent; on Day 11, the second induction was performed via tail vein injection (i.v.) of 35 mg/kg of dimethyl benzanthracene (DMBA). On Day 14, blood samples were collected from the model-induced animals for hematological indicator detection. These parameters served as the pre-administration baseline. The enrolled animals were grouped before administration, and the model-induced animals were divided into the model control group, the Roxadustat group (10 mg/kg), and the compound A group (10 mg/kg), and the administration began.

Administration and parameter detection: Starting from Day 15, the vehicle or the corresponding drug was administered to the normal control group, the model control group, the Roxadustat group (10 mg/kg), and the compound A group (10 mg/kg). Hematological indicator detection was conducted on Day 21 and Day 28, i.e., the first and second weeks after administration, respectively, and the differences in hematological indicators were analyzed and compared.

### 2. Experimental Results

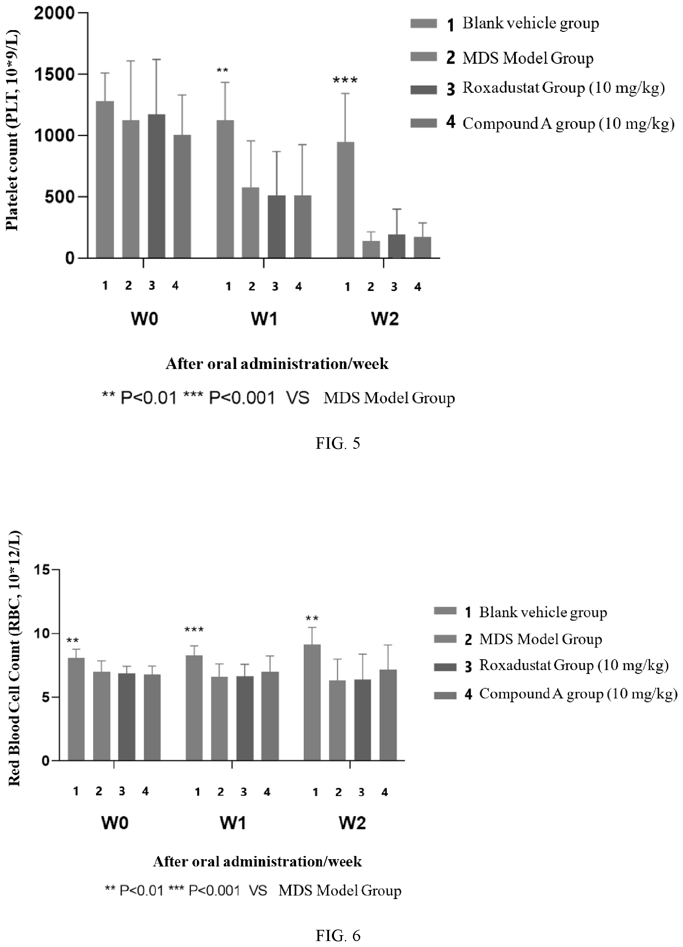

After injection of DMBA twice for induction, the data of red blood cells and platelets in the peripheral blood of rats decreased significantly. As reported in the literature, this indicated successful establishment of a rat model of myelodysplastic syndrome. From Day 14 after initial modeling, rats were received continuous administration for two weeks. Compared with the model group, the administration groups Roxadustat (10 milligrams per kilogram) and compound A (10 milligrams per kilogram) showed no significant changes in hematological parameters. Therefore, it was determined that neither drug demonstrated efficacy in this model. Details are shown in FIG. 5 and FIG. 6.

The aforementioned examples are the preferred embodiments of the present disclosure; however, the embodiments of the present disclosure are not limited thereto. Any other changes, modifications, substitutions, combinations, or simplifications made without departing from the essence of protection and principles of the present disclosure shall be equivalent substitutions and be included in the scope of protection of the present disclosure.

## Claims

1. Use of a compound in preparation of a medicament for treating anemia in a patient with a non-myeloid malignancy who is undergoing chemotherapy, wherein the compound is *N*-[7-hydroxy-5-(2-phenylethyl)[1,2,4]triazolo[1,5-a]pyridine-8-carbonyl]glycine.

2. Use of a pharmaceutical composition comprising a compound in preparation of a medicament for treating anemia in a patient with a non-myeloid malignancy who is undergoing chemotherapy, wherein the compound is *N*-[7-hydroxy-5-(2-phenylethyl)[1,2,4]triazolo[1,5-a]pyridine-8-carbonyl]glycine, and the pharmaceutical composition comprises one or more excipients.

3. The use according to claim 1 or 2, wherein the daily dose is about 1 mg to about 30 mg.

4. The use according to claim 1 or 2, wherein the patient is undergoing platinum-based myelosuppressive chemotherapy.

5. The use according to claim 1 or 2, wherein the patient is undergoing non-platinum-based myelosuppressive chemotherapy.

6. The use according to claim 1 or 2, wherein the chemotherapy is myelosuppressive chemotherapy, and a myelosuppressive chemotherapy agent comprises:
Atezolizumab, Bevacizumab, Reslizumab, Cetuximab, Denosumab, Durvalumab, Envafolimab, Inetetamab, Pembrolizumab, Penpulimab, Tislelizumab, Sintilimab, Toripalimab;
Gefitinib, Cabozantinib, Pyrotinib, Apatinib;
Carboplatin, Cisplatin, Lobaplatin, Nedaplatin, Oxaliplatin;
Paclitaxel, Docetaxel, Capecitabine, Gemcitabine, Cyclophosphamide, Epirubicin, Eribulin, Etoposide, Fluorouracil, Fulvestrant, Irinotecan, Doxorubicin, Cantharidin, Oteracil, Pemetrexed, Pyridoxine, Tamoxifen, Tegafur, and Vinorelbine.

7. The use according to claim 1 or 2, wherein the non-myeloid malignancy comprises breast cancer, lung cancer, gastrointestinal cancer, gynecological cancer, pancreatic cancer, and head and neck cancer.
